(19) Europäisches Patentamt
European Patent Office
Office européen des brevets

(11) Publication number: 0 155 492
B1

(12) EUROPEAN PATENT SPECIFICATION

(45) Date of publication of patent specification: 13.12.89

(51) Int. Cl.$^4$: C 07 D 231/12

(21) Application number: 85101392.0

(22) Date of filing: 09.02.85

# (54) Preparation of 1-alkyl-3,5-diphenylpyrazoles.

(30) Priority: 05.03.84 US 585949

(43) Date of publication of application:
25.09.85 Bulletin 85/39

(45) Publication of the grant of the patent:
13.12.89 Bulletin 89/50

(84) Designated Contracting States:
AT BE CH FR GB IT LI LU NL SE

(56) References cited:
DE-A-2 425 979
US-A-4 325 962
ANGEWANDTE CHEMIE, vol. 89, 1977, Weinheim; E.V. DEHMLOW "Fortschritte der Phasentransfer-Katalyse", pages 521-533
E.V.Dehlow-Phase Transfer Catalysis, Verlag Chemie-1980, page 1-3

The file contains technical information submitted after the application was filed and not included in this specification

(73) Proprietor: AMERICAN CYANAMID COMPANY
1937 West Main Street P.O. Box 60
Stamford Connecticut 06904-0060 (US)

(72) Inventor: Maulding, Donald Roy c/o American Cyanamid Comp.
1937 West Main Street P.O. Box 60
Stamford Connecticut 06904-0060 (US)

(74) Representative: Wächtershäuser, Günter, Dr.
Tal 29
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 155 492 B1

Courier Press, Leamington Spa, England.

## Description

Background of the Invention

The present invention relates to a novel method for preparing substituted and unsubstituted 1-alkyl-3,5-diphenylpyrazole compounds by reacting a 3,5-diphenyl (substituted or unsubstituted) pyrazole with an excess of dialkyl sulfate, in the presence of aqueous alkali hydroxide. This reaction also contains a quaternary ammonium phase transfer catalyst and is carried out at temperatures of about 20°C to 75° for about 1 to 2 hours.

Other methods have been developed for the preparation of such substituted and unsubstituted 1-alkyl-3,5-diphenylpyrazole compounds, but these methods utilize solid inorganic alkali metal strong bases, U.S. patent 3,907,824, Garber, September 23, 1975. Furthermore, these solid inorganic alkali metal base methods require anhydrous reaction conditions for several reasons. First, these anhydrous conditions ensure optimum reaction and ensure adequate storage conditions for the strong bases. Secondly, these anhydrous conditions are better suited for the careful control of the very exothermic resulting reaction.

Attempts have been made to prepare the 1-alkyl-3,5-diphenylpyrazoles of the present invention using aqueous bases without utilizing quaternary ammonium phase transfer catalysts (See Example 10 in Table II), but these attempts have resulted in an incomplete N-monomethylation reaction, with the remainder of substantial quantities of unreacted 3,5-diphenylpyrazoles.

The present invention provides a method for the preparation of a compound having the formula

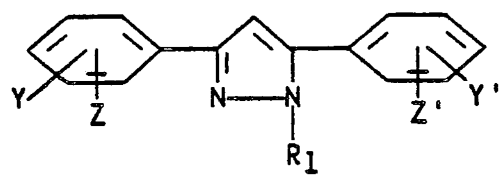

wherein $R_1$ is alkyl $C_1$—$C_4$ and Y, $Y^1$, Z and $Z^1$ each represent a member selected from the group consisting of hydrogen, nitro, halogen, alkyl $C_1$—$C_4$, haloalkyl $C_1$—$C_4$ containing 1 to 4 halogen groups, and alkoxy $C_1$—$C_4$, said method comprising: reacting a compound of the formula,

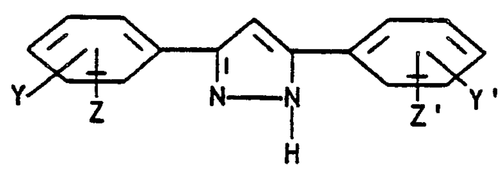

wherein Y, $Y^1$, Z and $Z^1$ are as defined above; with 1.0 to 1.50 molar equivalents of an alkylating agent represented by the formula $(R_1)_mQ$, wherein $R_1$ is as defined above and Q is a member selected from the group consisting of an organic sulfate, hydrogen sulfate, benzene sulfonate, $C_1$—$C_4$ alkoxybenzene sulfonate, phosphate, alkane sulfonate $C_1$—$C_4$, and *m* is 1, 2 or 3; in the presence of an aqueous, inorganic alkali metal base, characterized in that said reaction is conducted in the presence of 1.0 to 2.5 molar equivalents of aqueous inorganic alkali metal base and a nonaqueous, inert, organic solvent in the presence of a quaternary ammonium phase transfer catalyst, at a temperature between 20°C and 75°C; and a 1-alkyl-3,5-diphenylpyrazole is recovered.

Summary of the Invention

The present invention, therefore, relates to a novel method for preparing substituted and unsubstituted 1-alkyl-3,5-diphenylpyrazole compounds having structural formula (I),

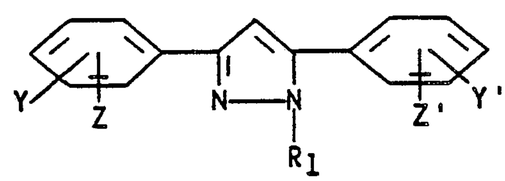

(I)

wherein $R^1$ is $C_1$ to $C_4$ alkyl and Y, $Y^1$, Z and $Z^1$ each represents hydrogen, nitro, halogen, $C_1$ to $C_4$ alkyl, $C_1$ to $C_4$ haloalkyl and $C_1$ to $C_4$ alkoxy. These formula (I) compounds are prepared by reacting a 3,5-diphenyl (substituted or unsubstituted) pyrazole, dissolved in an inert organic solvent, with an excess, about 1.0 to 1.5 moles, of an alkylating agent such as dialkylsulfate, in the present of about 1.0 to 2.5 moles preferably about 1.15 to 2.10 moles of a 40 to 60% (preferably 50%) aqueous inorganic alkali metal base such as an

alkali hydroxide and about 1.0% to 10.0% of a quaternary ammonium phase transfer catalyst. This reaction is carried out at temperatures of about 20°C to 75°C for a time period of about one (1) to two (2) hours.

Accordingly, it is an object of the present invention to provide a novel method for preparing said formula (I) compounds utilizing an aqueous base in the presence of a quaternary ammonium phase transfer catalyst, thereby avoiding the drawbacks of the prior art solid base-anhydrous systems.

It is a further object of the present invention to provide a method for preparing formula (I) compounds that result in enhanced N-mono-methylation reactions without leaving substantial quantities of unreacted 3,5-diphenylpyrazoles.

These and other objects of the present invention will become apparent by the more detailed description that follows.

Description of the Invention

In addition to the formula (I) compounds disclosed above, the process of the present invention for formulating such compounds includes other reactive components.

Solvents which can be used in the preparation of the 1-alkyl-3,5-diphenylpyrazoles include nonaqueous, inert organic solvents. Preferably, these solvents are selected from aromatic hydrocarbons, aliphatic hydrocarbons of $C_6$ to $C_{10}$ and halocarbons. Preferred aromatic hydrocarbons include benzene, toluene, xylene or mixtures thereof. Preferred aliphatic hydrocarbons suitable for the solvents of the present invention include hexane and heptane, and some useful halocarbons are ethylenedichloride, methylene chloride and mono- or dichlorobenzene.

Suitable aqueous inorganic alkali metal bases include sodium hydroxide and potassium hydroxide.

The present method also includes quaternary ammonium phase transfer catalysts, represented by structural formula (II),

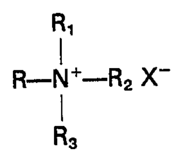

(II)

wherein $R^1$, $R^2$, $R^3$ are $C_1$ to $C_{18}$ alkyl, $C_1$ to $C_{18}$ alkenyl, $C_1$ to $C_{18}$ haloalkyl, benzyl, ethylhydroxyl, and $X^-$ is bromine, chlorine, methyl sulfate or bisulfate; or a dialkylpyrazolium compound wherein X is as previously defined.

The reaction of the present invention may be illustrated by the following flow diagram:

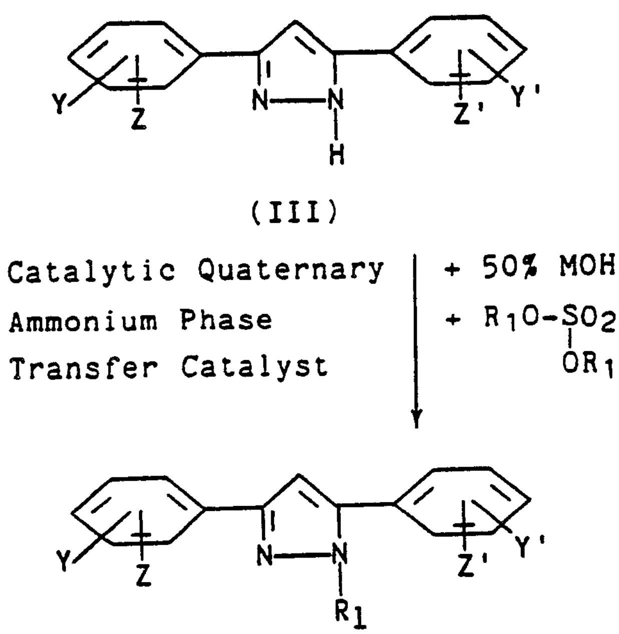


(III)

(I)

wherein $R_1$, Y, $Y^1$, Z, $Z^1$ and the quaternary ammonium phase transfer catalyst are as previously described. The present invention is of particular importance in the manufacture of 1-methyl-3,5-diphenylpyrazole and the conversion thereof to 1,2-dimethyl-3,5-diphenylpyrazolium methyl sulfate. The latter compound is a highly effective herbicidal agent. Moreover, said compound is especially useful for the selective control of wild oats in the presence of a wide variety of small grains, such as barley, rape, wheat and rye. The process

of the present invention results in the preparation of good (over 95%) yields of 1-methyl-3,5-diphenylpyrazole from 3,5-diphenylpyrazole and good purities (greater than 96%).

The methylation reaction is complete within about one to two hours with very little dimethylation occurring. After washing with aqueous base and water, the solvent, preferably xylene, containing essentially pure 1-methyl-3,5-diphenylpyrazole is concentrated to about 25% of its original volume. This concentrated solution is then directly used for the preparation of the 1,2-dimethyl-3,5-diphenylpyrazolium methyl sulfate salt.

The 1-methyl-3,5-diphenylpyrazole-xylene solution obtained from the above described reaction is mixed with ethylene dichloride in amounts approximately equivalent to the amount of xylene remaining in the concentrated pyrazole-xylene solution. The mixture is then heated to a temperature of about 50°C to 55°C. An equimolar amount, preferably a slight excess, about 1.0 to 1.5 moles, of the dialkyl sulfate such as dimethyl sulfate is added thereof. This mixture is stirred and heated to temperatures of about 90°C to 110°C for about four hours. The reaction mixture is cooled to 50°C and anhydrous triethylamine, equivalent to the excess of dialkyl sulfate used, is added.

This resulting mixture is stirred at 50°C for 30 minutes and then cooled to about 15°C, with the crystallized product being separated from the solvent. Any convenient means of separation may be employed, such as centrifugation, filtration, decantation or the like. The product cake is washed with an aromatic solvent, such as xylene; washed again with a lower ketone, such as acetone; and finally dried. Alternatively, the product cake may be dissolved in water, with the thus-prepared aqueous solution being directly used for the control of undesirable plant species.

In further characterizing this method, about 10% to 25% molar excess of dialkyl sulfate is added to the stirred mixture of 3,5-diphenylpyrazole (substituted or unsubstituted) dissolved in an inert organic solvent, such as xylene. Furthermore, about 15% to 105% molar excess of the 40% to 60% aqueous alkali hydroxide with quaternary ammonium phase transfer catalyst is used and at temperatures of about 23°C to 50°C.

The present invention is further illustrated by the following non-limiting examples.

Example 1

Preparation of 1-methyl-3,5-diphenylpyrazole

Benzyltriethylammonium chloride (1.14 g of 60% aqueous solution) was added to a mixture of 22.1 g (0.10 mol) of 3,5-diphenylpyrazole, 55 ml of xylene and 13.0 g of 50% aqueous NaOH. The stirred slurry was heated to 35°C and 15.7 g (0.124 mol) of dimethyl sulfate were added, dropwise, while under nitrogen. During this addition, the temperature was not allowed to exceed 55°C. The mixture was stirred at 45°C to 50°C for one and one-half hours, before 38 ml of water and 8 ml of 25% aqueous NaOH were added. This mixture was then stirred at 50° for an additional 30 minutes. After that, the xylene solution was separated from the mixture, and it was washed with water. Analysis of the xylene solution indicated that the yield of 1-methyl-3,5-diphenylpyrazole was 23.2 g (99.1%).

Examples 2—9

Preparation of 1-methyl-3,5-diphenylpyrazole

In order to compare the effects various reaction temperatures, reaction times, the stoichiometry of the excesses of aqueous sodium hydroxide or excesses of dimethyl sulfate, and the temperature ranges at which dimethyl sulfate is added to the system have, 1-methyl-3,5-diphenylpyrazole was prepared according to the procedure of Example 1, utilizing benzyl triethylammonium chloride as the phase transfer catalyst. The results of these variations are recorded in Table 1.

TABLE 1

Alkylation of 3,5-diphenylpyrazole

| Example number | Molar equivalents: Benzyltriethyl-ammonium chloride | Molar equivalents: Sodium hydroxide | Molar equivalents: Dimethyl sulfate | Dimethyl sulfate[a] additional temperature (°C) | Reaction: Temperature (°C) | Reaction: Time (hr) | Yield (%) 1-methyl-3,5-diphenylpyrazole |
|---|---|---|---|---|---|---|---|
| 2 | 0.02 | 1.15 | 1.10 | 50—55 | 26 | 1.0 | 93.0 |
| 3 | 0.03 | 1.15 | 1.10 | 51 | 35—40 | 2.0 | 97.0 |
| 4 | 0.03 | 1.15 | 1.15 | 31—35 | 35—40 | 2.0 | 96.2 |
| 5 | 0.03 | 1.15 | 1.10 | 53 | 45—50 | 1.5 | 100.0 |
| 6 | 0.03 | 1.30 | 1.23 | 53 | 45—50 | 1.5 | 96.3 |
| 7 | 0.03 | 1.63 | 1.23 | 58 | 45—50 | 1.5 | 98.3 |
| 8 | 0.03 | 2.05 | 1.23 | 50 | 45—50 | 1.5 | 98.2 |
| 9 | 0.03 | 1.63 | 1.12 | 47 | 45—50 | 1.5 | 100.0 |

[a]Maximum temperature attained after starting the addition at 30°C—35°C

Examples 10—21

Preparation of 1-methyl-3,5-diphenylpyrazole utilizing various quaternary ammonium phase transfer catalysts

In these examples, each quaternary ammonium phase transfer catalyst tested (0.5 mol% to 6.0 mol% based on pyrazole) was added to a mixture of 8.8 g (0.04 mol) of 3,5-diphenylpyrazole, 20 m of xylene and 3.7 g of 50% aqueous NaOH. Dimethyl sulfate (5.5 g, 0.044 mole) was added, while under nitrogen, at a rate so that the temperature did not exceed 55°C. The heterogeneous mixture was stirred at 25°C to 40°C for one to two hours and treated with 10 m of 6N $NH_4OH$ to destroy excess dimethylsulfate. The xylene layer was separated, washed with water and analyzed for 1-methyl-3,5-diphenylpyrazole. The results are summarized in Table II.

TABLE II

| Example | Phase-transfer catalyst | Mole % | Reaction Conditions: Temperature (°C) | Reaction Conditions: Time (hrs) | Yield (%) 1-methyl-3,5-diphenylpyrazole |
|---|---|---|---|---|---|
| 10 | None | 0.0 | 23 | 2.0 | 61.4 |
| 11 | 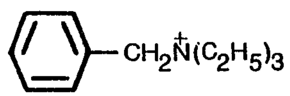 $Cl^-$ | 2.0 | 26 | 1.0 | 93.0 |
| 12 | | 3.0 | 35—40 | 2.0 | 97.0 |
| 13 | $(CH_3)_4N^+Cl^-$ | 2.0 | 23 | 1.0 | 70.7 |
| 14 | $C_{12}H_{25}-N^+(CH_3)(CH_2CH_2OH)_2Cl^-$ | 0.5 | 35—40 | 1.0 | 79.6 |
| 15 | $(CH_3)_3N^+CH_2CH_2Cl\ Cl^-$ | 2.0 | 24 | 1.0 | 71.2 |
| 16 | $C_{18}H_{37}N^+(CH_2CH_2OH)_2Cl^-$ | 1.4 | 23 | 1.0 | 87.9 |
| 17 | $(C_8H_{17})_3N^+Me\ Cl^-$ | 2.4 | 24 | 1.0 | 94.0 |
| 18 | | 2.4 | 35—40 | 1.0 | 96.0 |
| 19 | 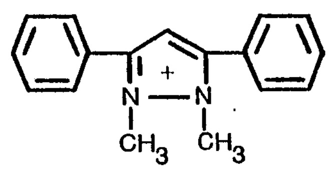 $CH_3OSO_3^-$ | 2.0 | 24 | 1.0 | 89.0 |
| 20 | | 5.0 | 23 | 2.0 | 97.6 |
| 21 | $(C_4H_9)_4N^+HSO_4^-$ | 2.0 | 28 | 2.0 | 91.2 |

## Claims

1. A method for the preparation of a compound having the formula,

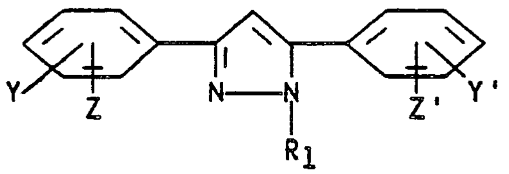

wherein $R_1$ is alkyl $C_1$—$C_4$ and Y, $Y^1$, Z and $Z^1$ each represent a member selected from the group consisting of hydrogen, nitro, halogen, alkyl $C_1$—$C_4$, haloalkyl $C_1$—$C_4$ containing 1 to 4 halogen groups, and alkoxy $C_1$—$C_4$, said method comprising: reacting a compound of the formula,

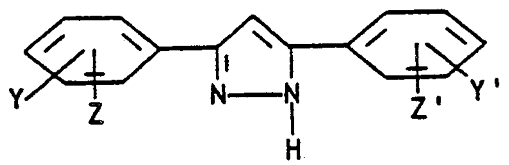

wherein Y, $Y^1$, Z and $Z^1$ are as defined above; with 1.0 to 1.50 molar equivalents of an alkylating agent represented by the formula $(R_1)_mQ$, wherein $R_1$ is as defined above and Q is a member selected from the group consisting of an organic sulfate, hydrogen sulfate, benzene sulfonate, $C_1$—$C_4$ alkoxybenzene sulfonate, phosphate, alkane sulfonate $C_1$—$C_4$, and *m* is 1, 2 or 3; in the presence of an aqueous, inorganic alkali metal base, characterized in that said reaction is conducted in the presence of 1.0 to 2.5 molar equivalents of aqueous inorganic alkali metal base and a nonaqueous, inert, organic solvent in the presence of a quaternary ammonium phase transfer catalyst, at a temperature between 20°C and 75°C; and a 1-alkyl-3,5-diphenylpyrazole is recovered.

2. A method according to Claim 1, wherein the molar equivalents of an aqueous inorganic, alkali metal base are 1.15 to 2.10.

3. A method according to Claim 2, wherein the alkylating agent is selected from the group consisting of a dialkyl sulfate, an alkyl hydrogen sulfate, and an alkyl toluene sulfonate, wherein each alkyl group contains from 1 to 4 carbon atoms; and the solvent is selected from the group consisting of an aromatic hydrocarbon, $C_6$—$C_{10}$ aliphatic hydrocarbon, 1,2-dichloroethane, methylene chloride, mono- or dichlorobenzene.

4. A method according to Claim 1 or 2 wherein 1% to 10% of a molar equivalent of a quaternary ammonium phase transfer catalyst having the structure,

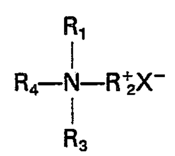

wherein $R_1$, $R_2$, $R_3$ and $R_4$ each represent a group selected from $C_1$—$C_{18}$ alkyl, $C_1$—$C_{18}$ alkenyl, $C_1$—$C_{18}$ haloalkyl, benzyl, ethylhydroxyl, and $X^-$ is bromine, chlorine, methyl sulfate or bisulfate; or a dialkylpyrazolium compound where X is defined as above, is employed.

5. A method according to Claim 1 or 2, wherein the quaternary ammonium phase transfer catalyst is benzyltriethyl ammonium chloride, tricapryl methyl ammonium chloride, 1,2-dimethyl-3,5-diphenyl-pyrazolium methyl sulfate, or mixtures thereof, at levels of 1% to 10% molar equivalent.

6. A method according to Claim 1 or 2, wherein $R_1$ is methyl; Y, $Y^1$, Z and $Z^1$ are each hydrogen; the alkylating agent is dimethyl sulfate; and the solvent is xylene.

7. A method according to Claim 4, wherein the base is 40% to 60%, by weight, of aqueous sodium hydroxide or potassium hydroxide.

8. A method according to Claim 5, wherein the reaction is carried out at a temperature range of 23°C to 50°C.

9. A method according to Claim 8, wherein the reaction is carried out at a temperature range of 35°C to 50°C.

**Patentansprüche**

1. Verfahren zür Herstellung einer Verbindung der Formel

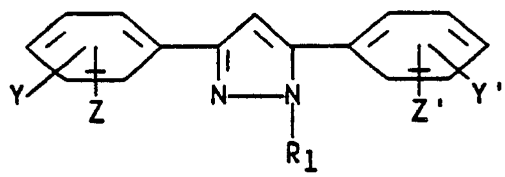

wobei $R_1$ für Alkyl-$C_{1-4}$ steht und Y, $Y^1$, Z und $Z^1$ jeweils einen Substituenten bedeuten, ausgewählt aus der Gruppe bestehend aus Wasserstoff, Nitro, Halogen, Alkyl-$C_{1-4}$, Halogenalkyl-$C_{1-4}$ enthaltend 1 bis 4 Halogengruppen, und Alkoxy-$C_{1-4}$, wobei das Verfahren umfaßt: Umsetzung einer Verbindung der Formel

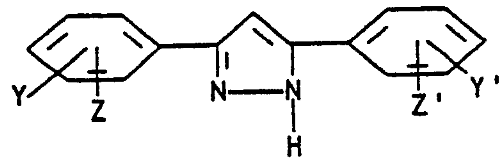

wobei Y, $Y^1$, Z und $Z^1$ wie oben definiert sind, mit 1,0 bis 1,50 molaren Äquivalenten eines Alkylierungsmittels der Formel $(R_1)_mQ$, wobei $R_1$ die oben angegebene Bedeutung hat und Q für einen Rest steht, ausgewählt aus der Gruppe bestehend aus einem organischen Sulfat, Hydrogensulfat, Benzolsulfonat, $C_{1-4}$-Alkoxybenzolsulfonat, Phosphat, Alkansulfonat-$C_{1-4}$ und *m* für 1, 2 oder 3 steht, in Gegenwart einer wässrigen, anorganischen Alkalimetallbase, dadurch gekennzeichnet, daß man die Umsetzung in Gegenwart von 1,0 bis 2,5 molaren Äquivalenten wäßriger, anorganischer Alkalimetallbase und einem nicht wäßrigen, inerten, organischen Lösungsmittel in Anwesenheit eines quaternären Ammoniumphasentransfer-Katalysators bei einer Temperatur zwischen 20°C und 75°C durchführt und ein 1-Alkyl-3,5-diphenylpyrazol isoliert.

2. Verfahren gemäß Anspruch 1, wobei die molaren Äquivalente einer wäßrigen, anorganischen Alkalimetallbase 1,15 bis 2,10 betragen.

3. Verfahren gemäß Anspruch 2, wobei das Alkylierungsmittel ausgewählt ist aus der Gruppe bestehend aus einem Dialkylsulfat, einem Alkylhydrogensulfat und einem Alkyltoluolsulfonat, wobei die jeweilige Alkylgruppe von 1 bis 4 Kohlenstoffatome enthält; und das Lösungsmittel ausgewählt ist aus der Gruppe bestehend aus einem aromatischen Kohlenwasserstoff, $C_{6-10}$-aliphatischem Kohlenwasserstoff, 1,2-Dichlorethan, Methylenchlorid, Mono- oder Dichlorbenzol.

4. Verfahren gemäß Anspruch 1 oder 2, wobei 1 bis 10% eines molaren Äquivalents eines quaternären Ammoniumphasentransfer-Katalysators mit der Struktur

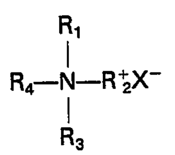

wobei $R_1$, $R_2$, $R_3$ und $R_4$ jeweils für eine Gruppe, ausgewählt aus $C_{1-18}$-Alkyl, $C_{1-18}$-Alkenyl, $C_{1-18}$-Halogenalkyl, Benzyl, Ethylhydroxyl, stehen und $X^-$ für Brom, Chlor, Methylsulfat oder Bisulfat steht; oder einer Dialkylpyrazoliumverbindung, wobei X die oben angegebene Bedeutung hat, eingesetzt wird.

5. Verfahren gemäß Anspruch 1 oder 2, wobei der quaternäre Ammoniumphasentransfer-Katalysator Benzyltriethylammoniumchlorid, Tricaprylammoniumchlorid, 1,2-Dimethyl-3,5-diphenylpyrazoliummethylsulfat oder Mischungen derselben ist, und zwar bei Niveaus von 1 bis 10% des molaren Äquivalents.

6. Verfahren gemäß Anspruch 1 oder 2, wobei $R_1$ für Methyl steht; Y, $Y^1$, Z und $Z^1$ jeweils Wasserstoff sind; das Alkylierungsmittel Dimethylsulfat ist; und das Lösungsmittel Xylol ist.

7. Verfahren gemäß Anspruch 4, wobei die Base 40 bis 60 Gew.-%iges wäßriges Natriumhydroxyd oder Kaliumhydroxyd ist.

8. Verfahren gemäß Anspruch 5, wobei die Umsetzung bei einer Temperatur im Bereich von 23°C bis 50°C durchgeführt wird.

9. Verfahren gemäß Anspruch 8, wobei die Umsetzung bei einer Temperatur im Bereich von 35°C bis 50°C durchgeführt wird.

**Revendications**

1. Procédé de préparation d'un composé de formule:

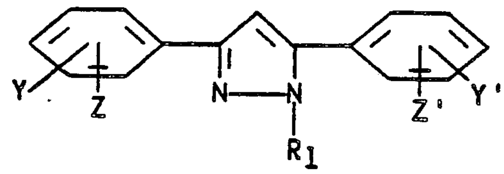

dans laquelle $R_1$ représente un groupe alkyle en $C_1$—$C_4$, et les groupes Y, $Y^1$, Z et $Z^1$, représentent chacun un groupe choisi parmi un atome d'hydrogène, un groupe nitro, un atome d'halogène, un groupe alkyle en $C_1$—$C_4$, un groupe haloalkyle en $C_1$—$C_4$ comportant de 1 à 4 groupes halogénés, et un groupe alkoxy en $C_1$—$C_4$; dans lequel on fait réagir un composé de formule:

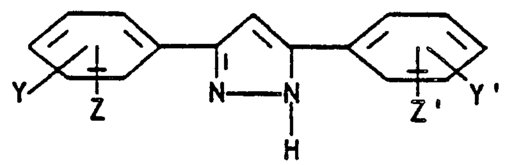

dans laquelle les groupes Y, $Y^1$, Z et $Z^1$ sont tels que définis ci-dessus; avec de 1,0 à 1,50 équivalents en moles d'un agent alkylant représenté par la formule $(R_1)_mQ$, dans laquelle $R_1$ est tel que défini ci-dessus et Q représente un groupe choisi parmi un groupe sulfate organique, hydrogénosulfate, benzène sulfonate, (alkoxy en $C_1$—$C_4$)benzène sulfonate, phosphate, (alcane en $C_1$—$C_4$)sulfonate, et *m* est égal à 1, 2 ou 3; en présence d'une base inorganique aqueuse dérivée de métal alcalin; caractérisé en ce que la réaction est effectuée en présence de 1,0 à 2,5 équivalents en moles d'une base inorganique aqueuse dérivée de métal alcalin, et d'un solvant organique inerte non aqueux, en présence d'un catalyseur de transfert de phase à base d'ammonium quaternaire, à une température de 20°C à 75°C; et on récupère un 1-alkyl-3,5-diphénylpyrazole.

2. Procédé selon la revendication 1, dans lequel le nombre d'équivalents en moles de base inorganique aqueuse dérivée de métal alcalin, varie de 1,15 à 2,10.

3. Procédé selon la revendication 2, dans lequel l'agent alkylant est choisi parmi un sulfate de dialkyle, un hydrogénosulfate d'alkyle et un toluène sulfonate d'alkyle, chaque groupe alkyle comportant de 1 à 4 atomes de carbone; et le solvant est choisi parmi un hydrocarbure aromatique, un hydrocarbure aliphatique en $C_6$ à $C_{10}$, le 1,2-dichloroéthane, le chlorure de méthylène, et le mono- ou le dichlorobenzène.

4. Procédé selon la revendication 1 ou 2, dans lequel on emploie de 1% à 10% d'un équivalent en mole d'un catalyseur de transfert de phase à base d'ammonium quaternaire, de structure:

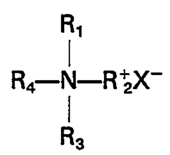

dans laquelle $R_1$, $R_2$, $R_3$ et $R_4$, représentent chacun un groupe choisi parmi un groupe alkyle en $C_1$—$C_{18}$, alkényle en $C_1$—$C_{18}$, haloalkyle en $C_1$—$C_{18}$, benzyle, éthoxy, et $X^-$ représente un atome de brome, de chlore, un groupe méthylsulfate ou bisulfate; ou d'un composé dérivé de dialkylpyrazolium dans lequel X est tel que défini ci-dessus.

5. Procédé selon la revendication 1 ou 2, dans lequel le catalyseur de transfert de phase à base d'ammonium quaternaire, est le chlorure de benzyltriéthyl ammonium, le chlorure de tricapryl méthyl ammonium, le méthyl sulfate de 1,2-diméthyl-3,5-diphénylpyrazolium ou des mélanges de ceux-ci, à raison de 1 à 10% d'équivalents en moles.

6. Procédé selon la revendication 1 ou 2, dans lequel $R_1$ représente un groupe méthyle; les groupes Y, $Y^1$, Z et $Z^1$ représentent chacun un atome d'hydrogène; l'agent alkylant est le sulfate de diméthyle; et le solvant est le xylène.

7. Procédé selon la revendication 4, dans lequel la base est une solution aqueuse d'hydroxyde de sodium ou de potassium à 40% jusqu'à 60% en poids.

8. Procédé selon la revendication 5, dans lequel la réaction est effectuée à une température de 23°C à 50°C.

9. Procédé selon la revendication 8, dans lequel la réaction est effectuée à une température de 35°C à 50°C.